# EUROPEAN PATENT SPECIFICATION

(11) **EP 2 376 069 B1**
(45) Date of publication and mention of the grant of the patent: **20.03.2019**
(21) Application number: 09796382.1
(22) Date of filing: 21.12.2009
(51) Int. Cl.: A61K 35/12, C12N 5/0775, A61K 9/50

(54) **MICROPARTICLES COMPRISING ADIPOSE STEM CELLS**
ZUSAMMENSETZUNGEN MIT ADIPÖSEN STAMMZELLEN
Compositions comprenant des cellules souches adipeuses

(30) Priority: 19.12.2008 EP 08382081
(43) Date of publication of application: 19.10.2011
(73) Proprietor: Cellerix, S.A., 28760 Tres Cantos - Madrid (ES)
(72) Inventor: ZANOTTI ACERO, Carlo, E-28760 Tres Cantos - Madrid (ES); MEDINA ALONSO, Jesús, E-28760 Tres Cantos - Madrid (ES); OROFINO VEGA, Javier, 28760 Tres Cantos - Madrid (ES)
(74) Representative: ABG Intellectual Property Law, S.L.
(86) International application number: PCT/EP2009/067660
(87) International publication number: WO 2010/070141

(56) References cited:
- WO-A-2008/143884
- US-A1- 2005 118 230
- DATABASE WPI Week 200915 Thomson Scientific, London, GB; AN 2009-B33634 XP002590050 & CN 101 285 053 A (UNIV DALIAN SCI & ENG) 15 October 2008 (2008-10-15)
- MALAFAYA P B ET AL: "Chitosan particles agglomerated scaffolds for cartilage and osteochondral tissue engineering approaches with adipose tissue derived stem cells" JOURNAL OF MATERIALS SCIENCE: MATERIALS IN MEDICINE, KLUWER ACADEMIC PUBLISHERS, BO, vol. 16, no. 12, 1 December 2005 (2005-12-01), pages 1077-1085, XP019399747 ISSN: 1573-4838
- ZHANG X ET AL: "In vivo adipogenesis by transplantation of adipose derived stromal cells on injectable scaffold" JOURNAL OF ALLOYS AND COMPOUNDS 20090512 ELSEVIER LTD; THE BOULEVARD GB, vol. 476, no. 1-2, 1 November 2008 (2008-11-01), pages 606-611, XP002530935 online
- ERICKSON GEOFFREY R ET AL: "Chondrogenic potential of adipose tissue-derived stromal cells in vitro and in vivo" BIOCHEMICAL AND BIOPHYSICAL RESEARCH COMMUNICATIONS, vol. 290, no. 2, 18 January 2002 (2002-01-18), pages 763-769, XP002530936 ISSN: 0006-291X
- MLADENOVSKA ET AL: "5-ASA loaded chitosan-Ca-alginate microparticles: Preparation and physicochemical characterization" INTERNATIONAL JOURNAL OF PHARMACEUTICS, ELSEVIER BV, NL, vol. 345, no. 1-2, 13 November 2007 (2007-11-13), pages 59-69, XP022342761 ISSN: 0378-5173
- WITTAYA-AREEKUL S ET AL: "Preparation and in vitro evaluation of mucoadhesive properties of alginate/chitosan microparticles containing prednisolone" INTERNATIONAL JOURNAL OF PHARMACEUTICS, ELSEVIER BV, NL, vol. 312, no. 1-2, 7 April 2006 (2006-04-07), pages 113-118, XP025113330 ISSN: 0378-5173 [retrieved on 2006-04-07]

## Description

### FIELD OF THE INVENTION

The present invention relates to adipose stem cells, methods for preserving said cells, kits comprising said preserved cells and uses of said compositions in the therapy of diseases.

### BACKGROUND OF THE INVENTION

STEM CELL THERAPY Mesenchymal stem cells (MSCs) are multipotent adult stem cells capable of differentiation into mesenchymal-type cells (adipocytes, osteoblasts and chondrocytes), but also myocytes, neurons, endothelial cells, astrocytes and epithelial cells. Although first reported in the normal adult bone marrow (BM-MSC), MSCs can also be obtained from other sources, such as umbilical cord blood, peripheral blood and adipose tissue.

The adipose tissue is a source of MSCs referred to as human adipose-derived mesenchymal stem cells (hASC), which can be isolated from liposuctioned fat tissue and expanded over a long time in culture. hASCs share some features with their counterpart in marrow, such as their differentiation potential, low immunogenicity and the ability to suppress immune responses. Recent studies comparing both cell types have reported differences at transcriptional and proteomic levels, suggesting that hASC and BM-MSC, while sharing similarities, are in fact quite different. The specific mechanisms underlying hASCs-mediated immunosuppression have so far been poorly studied. Recently, it has been reported that hASCs may inhibit lymphocyte proliferation by a mechanism that requires, at least in part, the release of PGE2. However, these studies did not provide information regarding (i) other cellular or soluble factors involved in the mechanism of immunosuppression, (ii) the immunosuppressive effect on isolated T cell subsets, or (iii) the phenotypic changes in both hASCs and PBMCs upon co-culture.

These biological abilities make MSCs, including hASCs, an interesting tool for cellular therapy and regeneration. This is further supported by studies showing that BM-MSCs alleviate allograft rejection, graft-versus-host disease, experimental autoimmune encephalomyelitis, collagen-induced arthritis and autoimmune myocarditis. Moreover, it has been recently reported that mouse ASCs (mASCs) were very efficient in protecting against graft-versus-host disease after allogeneic transplantation in an in vivo mouse model. In addition, MSCs are being used in several clinical trials with a focus on their immunomodulatory and antiinflammatory capacities. Adipose stem cells therapies appear promising in the treatment of a wide variety of diseases ranging from tissue regeneration to immune and/or inflammatory diseases. Although methods are available for treating these diseases, many current therapies provide less than adequate results, and carry the risk of significant side effects. Among new emergent therapeutic strategies, those based on cell therapy appear to constitute a potentially useful tool for treating a large number of diseases. Thus, a great effort is currently being made by researchers in order to achieve said aim.

AUTOIMMUNE DISEASES Autoimmune diseases are caused when the body's immune system, which is meant to defend the body against bacteria, viruses, and any other foreign product, malfunctions and produces a pathological response against healthy tissue, cells and organs.

T cells and macrophages provide beneficial protection, but can also produce harmful or deadly immunological responses. Autoimmune diseases can be organ specific or systemic and are provoked by different pathogenic mechanisms. Systemic autoimmune diseases involve polyclonal B cell activation and abnormalities of immunoregulatory T cells, T cell receptors and MHC genes. Examples of organ specific autoimmune diseases are diabetes, hyperthyroidism, autoimmune adrenal insufficiency, pure red cell anemia, multiple sclerosis and rheumatic carditis. Representative systemic autoimmune diseases include systemic lupus erythematosus, chronic inflammation, Sjogren's syndrome, polymyositis, dermatomyositis and scleroderma.

Current treatment of autoimmune diseases involves administering immunosuppressive agents such as cortisone, aspirin derivatives, hydroxychloroquine, methotrexate, azathioprine and cyclophosphamide or combinations thereof. The dilemma faced when administering immunosuppressive agents, however, is the more effectively the autoimmune disease is treated, the more defenseless the patient is left to attack from infections, and also the more susceptible for developing tumors. Thus, there is a great need for new therapies for the treatment of autoimmune diseases.

INFLAMMATORY DISORDERS Inflammation is a process by which the body's white blood cells and secreted factors protect our bodies from infection by foreign substances, such as bacteria and viruses and is a common process in autoimmune diseases. Secreted factors known as cytokines and prostaglandins control this process, and are released in an ordered and self-limiting cascade into the blood or affected tissues. In general, the current treatments for chronic inflammatory disorders have a very limited efficiency, and many of them have a high incidence of side effects or cannot completely prevent disease progression So far, no treatment is ideal, and there is no cure for these type of pathologies. Thus, there is a great need for new therapies for the treatment of inflammatory disorders.

ASC STORAGE Although ASC show great promise in treating disorders, the use of ASC is limited by their shelf life. The shelf life of ASC stored in culture media at room temperature is limited, and currently the only method for long term storage of ASC is cryopreservation. Cryopreservation of stem cells is costly, requires specialized equipment, and significantly affects stem cell viability. Accordingly there is a long felt need in the art for an improved method of ASC storage.

### SUMMARY OF THE INVENTION

The present invention provides compositions comprising of a polymer network in which adipose stem cells are suspended, as well as methods for making said compositions, kits comprising said compositions and the use thereof in the therapy of diseases.

### DEFINITIONS

In order to facilitate the understanding of the present description, the meaning of some terms and expressions in the context of the invention will be explained below. Further definitions will be included along the description as necessary.

As used herein the term "microencapsulated ASC" shall be taken to mean a three dimensional structure having a greatest dimension between 25 µm and 500 µm and comprising of adipose derived stem cells and one or more adhesive agents.

The term "adhesive agent" shall be taken to mean any agent which holds together the adipose derived stem cells and any other components of a microencapsulated ASC.

The term "allogeneic" as used herein shall be taken to mean from different individuals of the same species. Two or more individuals are said to be allogeneic to one another when the genes at one or more loci are not identical.

The term "autologous" as used herein shall be taken to mean from the same individual.

The expression "flow focusing", as used herein refers to a capillary focussing system which combines the application of hydrodynamic forces and a specific geometry of the system to generate fragments with the required properties. The fragments may be treated under conditions adequate for solidification, giving particles of nano and micrometric size, with spherical shape and a very narrow and reproducible size distribution.

The term "autoimmune disease" refers to a condition in a subject characterized by cellular, tissue and/or organ injury caused by an immunological reaction of the subject to its own cells, tissues and/or organs. Illustrative, non-limiting examples of autoimmune diseases which can be treated with the immunomodulatory cells of the invention include alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, Meniere's disease, mixed connective tissue disease, multiple sclerosis, type 1 or immune-mediated diabetes mellitus, myasthenia gravis, pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, sarcoidosis, scleroderma, progressive systemic sclerosis, Sjogren's syndrome, Good pasture's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, Wegener's granulomatosis, Anti-Glomerular Basement Membrane Disease, Antiphospholipid Syndrome, Autoimmune Diseases of the Nervous System , Familial Mediterranean Fever, Lambert-Eaton Myasthenic Syndrome, Sympathetic Ophthalmia, Polyendocrinopathies, Psoriasis etc.

The term " inflammatory disease" refers to a condition in a subject characterized by inflammation, eg, chronic inflammation Illustrative, non-limiting examples of inflammatory disorders include, but are not limited to, Celiac Disease, rheumatoid arthritis (RA), Inflammatory Bowel Disease (IBD), asthma, encephalitis, chronic obstructive pulmonary disease (COPD), inflammatory osteolysis, allergic disorders, septic shock, pulmonary fibrosis (e g , idiopathic pulmonary fibrosis), inflammatory vasculitis (eg, polyarteritis nodosa, Wegner's granulomatosis, Takayasu's arteritis, temporal arteritis, and lymphomatoid granulomatosus), post-traumatic vascular angioplasty (eg, restenosis after angioplasty), undifferentiated spondyloarthropathy, undifferentiated arthropathy, arthritis, inflammatory osteolysis, chronic hepatitis, and chronic inflammation resulting from chronic viral or bacteria infections.

The term "subject" refers to an animal, preferably a mammal including a non-primate (eg, a cow, pig, horse, cat, dog, rat, or mouse) and a primate (eg, a monkey, or a human). In a preferred embodiment, the subject is a human.

As used herein, "negative" or "-" as used with respect to cell surface markers shall be taken to mean that, in a cell population, less than 20%, 10%, preferably 9%, 8%, 7%, 6%, 5%, 4%, 3%, 2%, 1 % or none of the cells express said marker. Expression of cell surface markers may be determined for example by means of flow cytometry for a specific cell surface marker using conventional methods and apparatus (for example a Becton Dickinson FACS Calibur system used with commercially available antibodies and standard protocols known in the art).

As used herein the term adipose stem cell (also referred to herein as "ASC") shall be taken to mean a multipotent cell type originally derived from adipose tissue. The term "stem cell" shall be taken to mean a cell that, by successive divisions, can give rise to specialised cells. Multipotent stem cells can give rise to multiple types of specialized cells.

As used herein the term "embedded" (also referred herein as evenly dispersed or suspended) when referring to the adipose stem cells with respect to three-dimensional structure shall be taken to mean that a substantial percentage of the cells are enclosed in the surrounding mass of the three dimensional structure. This spatial organization usually occurs when the three-dimensional structure is formed by cross-linking a suspension of the cells in an adhesive agent and is to be distinguished from a three-dimensional structure obtained by seeding the wherein the cells on the structure after the three-dimensional structure is formed. The term "substantial percentage" when referring to the cells which are enclosed in the three dimensional structure shall be taken to mean that at least 5%, at least 10%, at least 20%, at least 30%, at least 40%, at least 50%, at least 60%, at least 70%, at least 80%, at least 90%,at least 91%,%, at least 92% at least 93%, at least 94%, at least 95%, at least 96%, at least 97%, at least 98%, at least 99% or more of the cells are not accessible to reagents which size is larger than the average pore size of the three dimensional structure. For instance, the percentage of cells which can be stained with Trypan Blue can be used as an indicator of the number of cells which are on the surface of the three dimensional structure or enclosed/embedded within the three dimensional structure.

As used herein, the expression "significant expression" or its equivalent terms "positive" and "+" when used in regard to a cell surface marker shall be taken to mean that, in a cell population, more than 20%, preferably, 30%, 40%, 50%, 60%, 70%, 80%, 90% or all of the cells express said marker.

Expression of cell surface markers may be determined for example by means of flow cytometry for a specific cell surface marker using conventional methods and apparatus (for example the Becton Dickinson FACS Calibur system system used with commercially available antibodies and standard protocols known in the art) that show a signal for a specific cell surface marker in flow cytometry above the background signal using conventional methods and apparatus (for example a Becton Dickinson FACS Calibur system used with commercially available antibodies and standard protocols known in the art). The background signal is defined as the signal intensity given by a non-specific antibody of the same isotype as the specific antibody used to detect each surface marker in conventional FACS analysis. For a marker to be considered positive the specific signal observed is stronger than 20%, preferably, 30%, 40%, 50%, 60%, 70%, 80%, 90%, 500%, 1000%, 5000%, 10000% or above, than the background signal intensity using conventional methods and apparatus (for example a Becton Dickinson FACS Calibur system used with commercially available antibodies and standard protocols known in the art).

As used herein, the terms "treat", "treatment" and "treating" when used directly in reference to a patient or subject shall be taken to mean the amelioration of one or more symptoms associated with a disorder including, but not limited to, an inflammatory disorder, an autoimmune disease or an immunologically mediated disease including rejection of transplanted organs and tissues, wherein said amelioration results from the administration of the immunomodulatory cells of the invention, or a pharmaceutical composition comprising thereof, to a subject in need of said treatment.

### DETAILED DESCRIPTION OF THE INVENTION

### Compositions of the invention

The authors of the present invention have observed that the encapsulation of ASC in a cross-linked polymeric matrix results in a surprising increase in the shelf-life of the cells (as much as 3-fold) when compared to conventional storage methods. Thus, in one aspect, the invention provides a three dimensional structure having a greatest dimension between 25 µm to 500 µm and comprising adipose derived stem cells and one or more adhesive agents. The adhesive agent is a first cross-linked polymeric network, wherein the first cross- linked polymeric network is alginate, further comprising a second cross-linked polymeric network wherein said second cross-linked polymeric network encapsulates the first cross-linked network and wherein the second cross- linked polymeric network is chitosan. The structure of the invention is hereinafter also referred to as "microencapsulated ASC".

It is particularly preferred that said adhesive agent is a gel. Particularly preferred is a polymer gel comprising of an elastic cross-linked polymer network expanded in volume by a fluid.

In one embodiment said ASC are fully or in part encapsulated within or by said network. It is particularly preferred that the adipose derived stem cells are embedded within the polymeric network and not on the surface of the polymeric microparticle. In a further embodiment said ASC may be located fully or in part on the surface of said crosslinked polymeric network. The microencapsulated ASC further comprises a second crosslinked polymeric network enclosing said first crosslinked polymeric network comprising ASC, wherein the second crosslinked polymeric network is chitosan.

The three dimensional structures of the microencapsulated ASC have a greatest dimension between 25 µm and 500 µm, preferably between 80 µm and about 200 µm. It is also preferred that said structure is spherical, conical, cylindrical, tubular, ovoid, cuboid, rectangular or irregular in form. It is also preferred that the ASC are substantially evenly dispersed within said structure. It is also preferred that said ASC are suspended within said structure. The first cross-linked polymeric network comprises alginate.

In principle, any alginate capable of forming an hydrogel is suitable for use in the compositions of the invention. Thus, the compositions may comprise alginates formed predominantly by manuronic acid (M blocks), by glucuronic acid moieties (G blocks) or by regions showing a mixture of manuronic acid and glucuronic acid (MG blocks). The percentage and distribution of the different uronic acids varies according to the source of the alginate and contribute to the properties of the alginate. Suitable sources for obtaining alginates include, without limitation, *Laminaria hyperborea, Letonia nigrescens, Lessonia trabeculata, Durvillaea antarctica, Laminaria digitata, Eclonia maxima, Macrocystis pyrifera, Ascophyllum nodosum* and/or *Laminaria japonica* as well as mixtures of alginates from different sources combined so as to obtain the desired contents in GG, MM and GM blocks. GG blocks contribute to the rigidity of the hydrogel, whereas MM blocks show a great resistance to fracture. Thus, by selecting an adequate combination of alginate polymers, it is possible to obtain alginates showing an elasticity module adequate while at the same time providing solutions which a viscosity which is sufficiently low so as to allow an adequate handling and cell immobilization. Thus, the invention contemplates the use of GG alginates, MM alginates or mixtures thereof with a ration of GG to MM alginates of 90:10, 80:20, 70:30, 60:40, 50:50, 40:60, 30:70, 20:80 or 10:90.

Alternatively, the invention contemplates the use of alginates obtained from alginates which occur in nature which have been modified with enzymes capable of modifying the alginate building blocks. Thus, it is possible to treat alginates with C5-epimerases, which are capable of converting M blocks in G blocks, as well as with AlgE4 from Azotobacter vinelandii, which converts M blocks in MG blocks. Additionally, it is possible to use alginates modified by different types of physical treatments, in particular, gamma rays, ultrasound and UV light as described by Wasikiewicz, J.M. et al. (Radiation Physics and Chemistry, 2005, 73:287-295). Preferred alginates for use in the present invention include alginates comprising 65-75% of G blocks and 25-35% of M blocks, such as FMC Protanal LF 10/60, and alginates comprising 35-45% of G blocks and 55-65% of M blocks, such as FMC Protanal LF 10/60 LS.

The microencapsulated ASC of the invention further comprise a second polymeric network which encloses the microencapsulated ASC. The second polymeric network is chitosan. As used herein, chitosan can be generally described as a deacetylated derivative of chitin, a high molecular weight and second most abundant natural biopolymer commonly found in the shells of marine crustaceans and cell walls of fungi. Chitosan is a linear polysaccharide, composed of glucosamine and N-acetyl glucosamine linked in a β(1l-4) manner; the glucosamine/N-acetyl glucosamine ratio being referred to as the degree of deacetylation. The chitosan may be shrimp shell chitosan (85% deacetylated). Chitosan is one example of a material that may be pH-crosslinked to form a hydrogel using the controlled release process. The sol-gel transition of chitosan is due to presence of the primary amine at the C-2 position of the glucosamine residues. At low pH, these amines are protonated and positively charged, and chitosan is a water-soluble cationic polyelectrolyte. At higher pH (above approximately 6.5), the chitosan amines become deprotonated and the polymer loses its charge and becomes insoluble. The formation of inter-polymer associations (e.g., liquid crystalline domains or network junctions) results in the formation of hydrogels.

The microencapsulated ASC of the present invention each preferably comprise from about 20 x 10⁶ to about 1 x 10⁶ adipose stem cells per milliliter of total volume. In a further preferred embodiment said three dimensional structures of the present invention each preferably comprise from about 11 x 10⁶ to about 5 x 10⁶ adipose stem cells per milliliter of total volume.

In a further aspect the microencapsulated ASC of the present invention further comprises an environment or agents that maintains or promotes cell viability. Examples of such agents are known to the person skilled in the art and include but are not limited to suitable culture medium. Such medium may comprise, Dulbecco's Modified Eagle's Medium (DMEM), antibiotics (for example, 100units/ml Penicillin and 100µg/ml Streptomycin), glutamine (e.g. at a concentration of about 2 mM), fetal bovine serum (FBS) e.g. at a concentration of about 2% to about 20%. It is within the skill of one in the art to modify or modulate concentrations of media and/or media supplements as necessary. Other commonly used agents that maintain or promote cell viability include sera such as but not limited to human serum, human serum albumin (HSA), FBS, bovine serum (BS), calf serum (CS), fetal calf serum (FCS), newborn calf serum (NCS), goat serum (GS), horse serum (HS), porcine serum, sheep serum, rabbit serum and rat serum (RS). Other commonly used agents that maintain or promote cell viability include antimicrobial agents such as but not limited to antibiotics or anti-mycotic compounds e.g. penicillin/streptomycin, amphotericin, ampicillin, gentamicin, bleomycin, hygromacin, kanamycin and mitomycin. In an alternative embodiment said agent may be a hormone such as but are not limited to, D-aldosterone, diethylstilbestrol (DES), dexamethasone, b-estradiol, hydrocortisone, insulin, prolactin, progesterone and somatostatin/human growth hormone (HGH).

In a further aspect the microencapsulated ASC of the present invention further comprisess an environment or agents that maintains the ASC in an undifferentiated form. Examples of such agents are known to the person skilled in the art.

Wherein the adhesive agent of the microencapsulated ASC of the present invention is a polymer gel it is particularly preferred that the fluid comprising said gel comprises at least about 10%, 20% by weight of agents(s) that maintains or promotes cell viability such as but not limited to those medias described herein.

With respect to the intended recipient, the ASC used in the method of the present invention may be of either allogeneic (donor) or autologous (subject) origin. In one embodiment of the method said ASC are of allogeneic origin.

The ASC may be derived from adipose tissue of any suitable origin, but is most preferably human in origin. It is preferred that said cells are obtained from non-pathological mammalian sources, preferably post-natal (e. g., rodent; primate). However, particularly preferred are subcutaneous adipose tissue or organ associated adipose tissue (for example but not limited to adipose associated with the heart, liver, kidneys or pancreas). The ASC are preferably characterized in that (i) they do not express markers specific for APCs; (ii) they do not express IDO constitutively; (iii) they express IDO upon stimulation with IFN-γ; and (iv) they present capacity to be differentiated into at least two cell lineages.

ASC Markers The ASC are preferably negative for at least one, two, three, four or preferably all of the following markers CD11b, CD11c, CD14, CD45, and HLAII, which are specific markers for APCs lineages.

Moreover, the ASC are preferably negative for at least one, two of, or preferably all of the following cell surface markers: CD31, CD34 and CD133. As used herein, "negative" with respect to cell surface markers means that, in a cell population comprising the ASC, less than 10%, preferably 9%, 8%, 7%, 6%, 5%, 4%, 3%, 2%, 1% or none of the cells show a signal for a specific cell surface marker in flow cytometry above the background signal, using conventional methods and apparatus (for example a Beckman Coulter Epics XL FACS system used with commercially available antibodies and standard protocols known in the art). In a particular embodiment, the ASC are characterised in that they express at least one, two, three, four, of or preferably all of the following cell surface markers: CD9, CD44, CD54, CD90 and CD105; i.e., the ASC are positive for at least one, two, three, four of and preferably all said cell surface markers (CD9, CD44, CD54, CD90 and CD105). Preferably, the ASC are characterised in that they have significant expression levels of at least one, two, three, four, of and preferably all of said cell surface markers (CD9, CD44, CD54, CD90 and CD105). As used herein, the expression "significant expression" means that, in a cell population comprising the ASC, more than 10%, preferably 20%, 30%, 40%, 50%, 60%, 70%, 80%, 90% or all of the cells show a signal for a specific cell surface marker in flow cytometry above the background signal using conventional methods and apparatus (for example a Beckman Coulter Epics XL FACS system used with commercially available antibodies and standard protocols known in the art). The background signal is defined as the signal intensity given by a non-specific antibody of the same isotype as the specific antibody used to detect each surface marker in conventional FACS analysis. Thus for a marker to be considered positive the specific signal observed is stronger than 10%, preferably 20%, 30%, 40%, 50%, 60%, 70%, 80%, 90%, 500%, 1000%, 5000%, 10000% or above, than the background signal intensity using conventional methods and apparatus (for example a Beckman Coulter Epics XL FACS system used with commercially available antibodies and standard protocols known in the art).

Optionally, the ASC are also negative for the cell surface marker CD106(VCAM-1),. Examples of such cells are certain populations of adipose tissue-derived stromal stem cells as described herein. Commercially available and known monoclonal antibodies against said cell-surface markers (e.g., cellular receptors and transmembrane proteins) can be used to identify the ASC.

Expression of IDO The ASC preferably do not express IDO constitutively, but they express IDO upon stimulation with IFN-γ. Said cells, upon stimulation with other pro-inflammatory mediators such us interleukin-1 (IL-1) used at a concentration of 3ng/ml, tumour necrosis factor-alphaTNF-α) used at a concentration of 50ng/ml, or the endotoxin LPS used at a concentration of 100ng/ml, did not induce IDO expression, as measured by conventional RT-PCR and Western Blot analysis. Stimulation with IFN-γ for example at 3ng/ml or higher can also induce expression of HLAII in the ASC to give a positive signal as defined herein for a cell surface marker. Said expression can be detected by those skilled in the art using any known technique that allows the detection of the expression of specific proteins. Preferably, said techniques are cell cytometry techniques.

ASC Differentiation The ASC preferably present the capacity to proliferate and be differentiated into at least two, more preferably three, four, five, six, seven or more cell lineages. Illustrative, non-limiting examples of cell lineages in which the ASC can be differentiated include osteocytes, adipocytes, chondrocytes, tenocytes, myocytes, cardiomyocytes, hematopoietic-supporting stromal cells, endothelial cells, neurons, astrocytes, and hepatocytes. ASC can proliferate and differentiate into cells of other lineages, and can be induced to do so by means of conventional methods. Methods of identifying and subsequently isolating differentiated cells from their undifferentiated counterparts can be also carried out by methods well known in the art.

The ASC are also capable of being expanded *ex vivo.* That is, after isolation, the ASC can be maintained and allowed to proliferate *ex vivo* in culture medium. Such medium is composed of, for example, Dulbecco's Modified Eagle's Medium (DMEM), with antibiotics (for example, 100units/ml Penicillin and 100µg/ml Streptomycin) or without antibiotics, and 2 mM glutamine, and supplemented with 2-20% fetal bovine serum (FBS). It is within the skill of one in the art to modify or modulate concentrations of media and/or media supplements as necessary for the cells used. Sera often contain cellular and non-cellular factors and components that are necessary for viability and expansion. Examples of sera include FBS, bovine serum (BS), calf serum (CS), fetal calf serum (FCS), newborn calf serum (NCS), goat serum (GS), horse serum (HS), porcine serum, sheep serum, rabbit serum, rat serum (RS), etc. Also contemplated is, if the ASC are of human origin, supplementation of cell culture medium with a human serum, preferably of autologous origin. It is understood that sera can be heat-inactivated at 55-65°C if deemed necessary to inactivate components of the complement cascade. Modulation of serum concentrations, withdrawal of serum from the culture medium can also be used to promote survival of one or more desired cell types. Preferably, ASC will benefit from FBS concentrations of about 2% to about 25%. In another embodiment, the ASC can be expanded in a culture medium of definite composition, in which the serum is replaced by a combination of serum albumin, serum transferrin, selenium, and recombinant proteins including but not limited to: insulin, platelet-derived growth factor (PDGF), and basic fibroblast growth factor (bFGF) as known in the art.

Many cell culture media already contain amino acids; however some require supplementation prior to culturing cells. Such amino acids include, but are not limited to, L-alanine, L- arginine, L-aspartic acid, L-asparagine, L-cysteine, L-cystine, L-glutamic acid, L-glutamine, L-glycine, and the like.

Antimicrobial agents are also typically used in cell culture to mitigate bacterial, mycoplasmal, and fungal contamination. Typically, antibiotics or anti-mycotic compounds used are mixtures of penicillin/streptomycin, but can also include, but are not limited to amphotericin (Fungizone®), ampicillin, gentamicin, bleomycin, hygromacin, kanamycin, mitomycin, etc.

Hormones can also be advantageously used in cell culture and include, but are not limited to, D-aldosterone, diethylstilbestrol (DES), dexamethasone, b-estradiol, hydrocortisone, insulin, prolactin, progesterone, somatostatin/human growth hormone (HGH), etc.

The maintenance conditions of the ASC can also contain cellular factors that allow cells to remain in an undifferentiated form. It is apparent to those skilled in the art that prior to differentiation, supplements that inhibit cell differentiation must be removed from the culture medium. It is also apparent that not all cells will require these factors. In fact, these factors may elicit unwanted effects, depending on the cell type.

### Methods for preparing the compositions of the invention

The invention further provides a method for obtaining a 25-500 µm microparticle of adipose stem cells comprising the steps of:
(i) combining a suspension of adipose stem cells and a solution of a cross-linkable polymer,
(ii) fragmenting the mixture obtained in step (i),
(iii) placing the fragments formed in step (ii) under conditions adequate for promoting the conversion of the cross-linkable polymer into a cross-linked polymeric network and
(iv) recovering the particles obtained in step (iii), wherein the cross-linkable polymer is alginate, the method further comprising placing the microparticles obtained in step (iv) in the presence of a second cross-linkable polymer under conditions adequate for the cross-linking of said polymer, wherein the second cross-linkable polymer is chitosan.

Step (i) of the method of the invention comprises the contacting or mixing a plurality of ASC with a polymer solution capable of forming hydrogels to form a mixture hereinafter referred to as a precursor solution and forming said a precursor solution into three dimensional microstructures. As used herein, the term "precursor solution" refers to a solution comprising one or more polymers and/or polymer precursors and ASC.

ASC for use in the compositions according to the present invention may be isolated using any method known in the art. In one embodiment this may comprise the steps of:
(i) preparing a cell suspension from a sample of adipose;
(ii) recovering the cells from said cell suspension;
(iii) incubating said cells in a suitable cell culture medium on a solid surface under conditions which allow cells to adhere to the solid surface and proliferate;
(iv) washing said solid surface after incubation to remove non-adhered cells;
(v) selecting the cells which after being passaged at least twice in such medium remain adhered to said solid surface; and
(vi) confirming that the selected cell population presents the phenotype of interest.

As used herein, the term "solid surface" refers to any material upon which the ASC can adhere. In a particular embodiment said material is a plastic material treated to promote the adhesion of mammalian cells to its surface, for example commercially available polystyrene plates optionally coated with poly-D-Lysine or other reagents.

Steps (i)-(vi) can be carried out by conventional techniques known by those skilled in the art. Briefly, the ASC can be obtained by conventional means from any suitable source of connective tissue from any suitable animal as discussed above. Typically, human adipose cells are obtained from living donors, using well-recognized protocols such as surgical or suction lipectomy. Indeed, as liposuction procedures are so common, liposuction effluent is a particularly preferred source from which the ASC can be derived. Thus, in a particular embodiment, the ASC are from the stromal fraction of human adipose tissue obtained by liposuction.

The tissue is, preferably, washed before being processed to separate the ASC from the remainder of the material. In one commonly used protocol, the sample of tissue is washed with physiologically-compatible saline solution (e.g., phosphate buffered saline (PBS)) and then vigorously agitated and left to settle, a step that removes loose matter (e.g., damaged tissue, blood, erythrocytes, etc) from the tissue. Thus, the washing and settling steps are generally repeated until the supernatant is relatively clear of debris. The remaining cells generally will be present in clumps of various sizes, and the protocol proceeds using steps gauged to degrade the gross structure while minimizing damage to the cells themselves. One method of achieving this end is to treat the washed lumps of cells with an enzyme that weakens or destroys bonds between cells (e.g., collagenase, dispase, trypsin, etc.). The amount and duration of such enzymatic treatment will vary, depending on the conditions employed, but the use of such enzymes is generally known in the art. Alternatively or in conjunction with such enzymatic treatment, the lumps of cells can be degraded using other treatments, such as mechanical agitation, sonic energy, thermal energy, etc. If degradation is accomplished by enzymatic methods, it is desirable to neutralize the enzyme following a suitable period, to minimize deleterious effects on the cells.

The degradation step typically produces a slurry or suspension of aggregated cells and a fluid fraction containing generally free stromal cells (e.g., red blood cells, smooth muscle cells, endothelial cells, fibroblast cells, and stem cells). The next stage in the separation process is to separate the aggregated cells from the ASC. This can be accomplished by centrifugation, which forces the cells into a pellet covered by a supernatant. The supernatant then can be discarded and the pellet suspended in a physiologically-compatible fluid. Moreover, the suspended cells typically include erythrocytes, and in most protocols it is desirable to lyse them. Methods for selectively lysing erythrocytes are known in the art, and any suitable protocol can be employed (e.g., incubation in a hyper -or hypotonic medium, by lysis using ammonium chloride, etc.). Of course, if the erythrocytes are lysed, the remaining cells should then be separated from the lysate, for example by filtration, sedimentation, or density fractionation.

Regardless of whether the erythrocytes are lysed, the suspended cells can be washed, re-centrifuged, and resuspended one or more successive times to achieve greater purity. Alternatively, the cells can be separated on the basis of cell surface marker profile or on the basis of cell size and granularity.

Following the final isolation and re-suspension, the cells can be cultured and, if desired, assayed for number and viability to assess the yield. Preferably, the cells will be cultured without differentiation, on a solid surface, using a suitable cell culture media, at the appropriate cell densities and culture conditions. Thus, in a particular embodiment, cells are cultured without differentiation on a solid surface, usually made of a plastic material, such as Petri dishes or cell culture flasks, in the presence of a suitable cell culture medium [e.g., DMEM, typically supplemented with 5-15% (e.g., 10%) of a suitable serum, such as fetal bovine serum or human serum], and incubated under conditions which allow cells to adhere to the solid surface and proliferate. After incubation, cells are washed in order to remove non-adhered cells and cell fragments. The cells are maintained in culture in the same medium and under the same conditions until they reach the adequate confluence, typically, about 70%, about 80% or about 90% cell confluence, with replacement of the cell culture medium when necessary. After reaching the desired cell confluence, the cells can be expanded by means of consecutive passages using a detachment agent such as trypsin and seeding onto a new cell culture surface at an appropriate cell density (usually 2,000-10,000 cells/cm²). Thus, cells are then passaged at least two times in such medium without differentiating, while still retaining their developmental phenotype, and more preferably, the cells can be passaged at least 10 times (e.g., at least 15 times or even at least 20 times) without losing developmental phenotype. Typically, the cells are plated at a desired density such as between about 100 cells/cm² to about 100,000 cells/cm² (such as about 500 cells/cm² to about 50,000 cells/cm², or, more particularly, between about 1,000 cells/cm² to about 20,000 cells/cm²). If plated at lower densities (e.g., about 300 cells/cm²), the cells can be more easily clonally isolated. For example, after a few days, cells plated at such densities will proliferate into an homogeneous population. In a particular embodiment, the cell density is between 2,000-10,000 cells/cm².

Cells which remain adhered to the solid surface after such treatment comprising at least two passages are selected and the phenotype of interest is analyzed by conventional methods in order to confirm the identity of the ASC as will be mentioned below. Cells which remain adhered to the solid surface after the first passage are from heterogeneous origin; therefore, said cells must be subjected to at least another passage. As a result of the above method, a homogeneous cell population having the phenotype of interest is obtained. The adhesion of cells to the solid surface after at least two passages constitutes a preferred embodiment of the invention for selecting the ASC. Confirmation of the phenotype of interest can be carried out by using conventional means.

Preferably said expansion is carried out by duplication or triplication of said population at least 1, at least 2, at least 3, at least 4, at least 5, at least 10, at least 15 or at least 20 times. In a further embodiment said expansion is carried over at least 1, at least 2, at least 3, at least 4, at least 5, at least 10, at least 15 or at least 20 passages.

Cell-surface markers can be identified by any suitable conventional technique, usually based on a positive/negative selection; for example, monoclonal antibodies against cell-surface markers, whose presence/absence in the cells is to be confirmed, can be used; although other techniques can also be used. Thus, in a particular embodiment, monoclonal antibodies against one, two, three, four, five, six, seven of or preferably all of CD11b, CD11c, CD14, CD45, HLAII, CD31, CD34 and CD133 are used in order to confirm the absence of said markers in the selected cells; and monoclonal antibodies against one, two, three, four, of or preferably all of CD9, CD44, CD54, CD90 and CD105 are used in order to confirm the presence thereof or detectable expression levels of, at least one of and preferably all of, said markers. Said monoclonal antibodies are known, commercially available or can be obtained by a skilled person in the art by conventional methods.

IFN-γ-inducible IDO activity in the selected cells can be determined by any suitable conventional assay. For example, the selected cells can be stimulated with IFN-γ and assayed for IDO expression; then conventional Western-blot analysis for IDO protein expression can be performed and IDO enzyme activity following IFN-γ stimulation of the selected cells can be measured by tryptophan-to-kynurenine conversion with for example via High Performance Liquid Chromatography (HPLC) analysis and photometric determination of kynurenine concentration in the supernatant as the readout. Since the ASC express IDO under certain conditions, any suitable technique which allows the detection of IDO activity following IFN-γ stimulation may be used for selecting the ASC. The amount of IDO produced depends on the number of cells per square centimetre, which is preferably at a level of 5000cells/cm² or more, but not limited to this concentration and the concentration of IFN-gamma, which ideally is 3ng/ml or more, but not limited to this concentration. The activity of IDO produced under the described conditions will result in a detectable production of kynurenine in the µM range after 24 hours or more.

The capacity of the selected cells to differentiate into at least two cell lineages can be assayed by conventional methods as known in the art.

ASC can be clonally expanded, if desired, using a suitable method for cloning cell populations. For example, a proliferated population of cells can be physically picked and seeded into a separate surface (or the well of a multi-well plate). Alternatively, the cells can be subcloned onto a multi-well plate at a statistical ratio for facilitating placing a single cell into each well (e.g., from about 0.1 to about 1 cell/well or even about 0.25 to about 0.5 cells/well, such as 0.5 cells/well). Of course, the cells can be cloned by plating them at low density (e.g., in a Petri dish or other suitable substrate) and isolating them from other cells using devices such as a cloning rings. The production of a clonal population can be expanded in any suitable culture medium. In any event, the isolated cells can be cultured to a suitable point when their developmental phenotype can be assessed.

It has been shown that *ex vivo* expansion of the ASC without inducing differentiation can be accomplished for extended time periods for example by using specially screened lots of suitable serum (such as fetal bovine serum or human serum). Methods for measuring viability and yield are known in the art (e. g., trypan blue exclusion). Any of the steps and procedures for isolating the cells of the cell population of the invention can be performed manually, if desired. Alternatively, the process of isolating such cells can be facilitated and/or automated through one or more suitable devices, examples of which are known in the art.

The formation of said microstructures is carried out by means of crosslinking or polymerizing said precursor solution. The polymer solution comprises one or more polymers and/or polymer precursors which are suitable for formation of a hydrogel by crosslinking and polymerisation. The polymeric network comprises alginate.

The amount of polymer in the polymer solution is sufficient to allow formation of hydrogels upon polymerization of the precursor solution. In one embodiment this amount is between about 70% and about 1% by weight of the polymer solution, alternatively said amount is between about 50% and about 1%, or between about 30% and about 1%, or between about 10% and about 1%, or between about 5% and 1%. In a particularly preferred embodiment of the method the percent of polymer in the polymer solution is between about 1.05% and about 1.25%.

It is preferred that said precursor solution comprises 20 x 10⁶ to about 1 x 10⁶ adipose stem cells per milliliter. In a further preferred embodiment said precursor solution preferably comprises from about 11 x 10⁶ to about 5 x 10⁶ adipose stem cells per milliliter. It is also preferred that said ASC are evenly distributed or suspended within the precursor solution.

The precursor solution may further comprise additional agents that maintains or promotes cell viability. Examples of such agents are known to the person skilled in the art and include but are not limited to suitable ASC culture medium. Such medium may comprise, Dulbecco's Modified Eagle's Medium (DMEM), antibiotics (for example, 100units/ml Penicillin and 100µg/ml Streptomycin), glutamine (e.g. at a concentration of about 2 mM), fetal bovine serum (FBS) e.g at a concentration of about 2% to about 20%. It is within the skill of one in the art to modify or modulate concentrations of media and/or media supplements as necessary. Other commonly used agents that maintain or promote cell viability include sera such as but not limited to human serum, FBS, bovine serum (BS), calf serum (CS), fetal calf serum (FCS), newborn calf serum (NCS), goat serum (GS), horse serum (HS), porcine serum, sheep serum, rabbit serum and rat serum (RS). Other commonly used agents that maintain or promote cell viability include antimicrobial agents such as but not limited to antibiotics or anti-mycotic compounds e.g. penicillin/streptomycin, amphotericin, ampicillin, gentamicin, bleomycin, hygromacin, kanamycin and mitomycin. In an alternative embodiment said agent may be a hormone such as but are not limited to, D-aldosterone, diethylstilbestrol (DES), dexamethasone, b-estradiol, hydrocortisone, insulin, prolactin, progesterone and somatostatin/human growth hormone (HGH).

In a further aspect the microencapsulated ASC of the present invention further comprises an environment or agents that maintains the ASC in an undifferentiated form. Examples of such agents are known to the person skilled in the art.

Step (ii) of the method of the invention comprises fragmenting the mixture obtained in step (i). The fragmentation step can be carried out by any means known in the art. The manufacture of microencapsulated cells is known to the person skilled in the art and includes the use of appropriately sized molds or dispersion of the precursor solution into fragments of the preferred size followed by polymerization. The formation of the fragments from the cell suspension can be carried out using any method known in the art such as using an electrostatic bead generator or flow focusing. However, in a preferred embodiment, the compositions of the invention are prepared using flow focusing.

The basic technology underlying flow focusing lies on a system for introduction of a first fluid inside a pressurized chamber by means of a second fluid. The first fluid can be a liquid or gas and the second fluid is a liquid. Preferably, both fluids are liquids different enough from each other to allow the generation of a stable microjet of the first fluid moving from the feeding end to the exit point of the pressurized chamber to the environment. Whereas both possible combinations gas-liquid and liquid-liquid are possible, the ASC are microencapsulated using a gas-liquid system wherein the first fluid is pressurized air and the second fluid is the ASC suspension comprising the polymeric compound prior to be cross-linked.

The formation of the microjet, its acceleration and finally the generation of the particles is based on the abrupt pressure drop associated with the sudden acceleration undergone by the first fluid pressurizing the chamber when going through the exit hole of the chamber. This causes a high pressure difference between both fluids, which originates the appearance of a high curve area in the surface of the second fluid close to the hole and obtaining a peak point from where a stationary microjet will flow if it is provided the same amount of liquid than the hole sucks. Thus, the fluid in the pressurized chamber surrounds the first fluid focussing it, generating a stable microjet whose diameter is much smaller than the diameter of the exit hole of the chamber preventing its clogging.

The skilled person will appreciate that one or more parameters of the flow focusing technique may be modified to optimize the resulting microparticles. By way of an example, it is possible to use focused cell suspension of different viscosities, flow rate of the focusing fluid and focused solution, feeding end diameter, size of the exit hole, pressure relationship, and the like. Preferred conditions for flow focusing according to the invention include 20 mbar pressure of the focusing gas and 65 mL/h of flow-rate for the focused cell suspension. Under these conditions, the average size of the resulting particles is of ca. 140 µm.

The system allows can be applied to almost every liquid (dynamic viscosities from 10-4 to 1 Kg.m⁻1s⁻¹), so the capillary microjet rising from the feeding tube is absolutely stable and the instabilities in the microjet dissociation process cannot develop upstream. Downstream the microjet breaks into fragments of regular size simply by capillary instability (e.g. Rayleigh "On the instability of jets" Proc. London Math. Soc, 4-13, 1878).

The microjet is generated inside the fluid in motion in a way that when the microjet breaks into similar fragments of the predicted size the emulsion is produced. After extraction/evaporation of the solvent by means of any of the already known methods we obtain particles of nano- and micrometric size with spherical shape and a very narrow and reproducible size distribution, as predicted by flow focussing theory.

Step (iii) comprises placing the particles under conditions adequate for the formation of cross-links within the cross-linkable polymer. The skilled person will appreciate that the cross-linking conditions will be different depending on the type of polymer which has been used. Thus, when the polymer is alginate, cross-linking is carried out by placing the fragments into a solution comprising divalent cations such as Ca²⁺, Sr²⁺, Ba²⁺ or polymeric cations such as poly-L-lysine. Gelling of alginate solutions is carried out essentially by the dyalisis/diffusion gelling method or by the internal gelling method. If desired, the bivalent ions are complexed so that they are slowly released from the complex depending under slightly acidic conditions. If the polymer is agarose, gelling takes place by decreasing temperature from an agarose solution at high temperature. If the polymer is chitosan, gelling takes place by modifying the pH of the solution.

Step (iv) of the method of the invention involves collecting the formed microparticles. The recovery of the microparticles can be carried out by any method known to the skilled person such as centrifugation, filtration, flotation and the like.

The microencapsulated ASC of the invention may be further contacted with an additional cross-linkable polymer under conditions adequate for the cross-linking of said polymer, thus forming a capsule which encloses the microencapsulated ASC. The second cross-linkable polymer is chitosan.

The microencapsulated ASC of the invention are preferably stored in an enclosed environment such as a sealed storage vessel. Suitable sealable containers for the storage of microencapsulated ASC are known in the art. Preferably the microencapsulated ASC within the sealed vessel are maintained in a gas environment. Wherein the microencapsulated ASC within the sealed vessel are maintained within a gas environment it is preferred that said environment comprises essentially of carbon dioxide or air (preferably of medicinal quality). Preferably the microencapsulated cells are maintained at a temperature of between about 4 degrees centigrade to about 25 degrees centigrade, in an alternative embodiment said microencapsulated cells may be stored at room temperature. Accordingly the present invention provides a sealed vessel containing microencapsulated cells of the present invention. In one embodiment of the method said container contains between about 10⁵ to about 10⁹ ASC. In one embodiment of the method said container contains between about 10⁵ to about 10⁸ ASC. In one embodiment of the method said container contains between about 10⁶ to about 10⁷ ASC.

In a preferred embodiment, the ASC contained within the microencapsulated cells may be dissociated therefrom by means of any suitable solvent or other agent. Preferably said agents are EDTA and/or sodium citrate dehydrate. Briefly the ASC may be dissociated by contacting the microencapsulated cells with an appropriate solvent or agent under conditions sufficient to dissolve, degrade or otherwise break down the polymers sufficient to allow the ASC to dissociate therefrom and to release the ASC. Optionally the solvent may be mixed or otherwise physically agitated to aid said dissociation. The dissociated ASC may then be isolated from the mixture by standards means known to the person skilled in the art such as centrifugation, filtration, flotation and the like.

In another embodiment, the invention relates to microparticles comprising the ASC which have been prepared using the method of the invention. Further particulars regarding the type of polymeric network comprising the microparticles, the optional second polymeric network have been described in detail when referring to the compositions of the invention and are equally applicable to the microparticles obtained by the method of the invention.

### Medical uses of the compositions of the invention

The three-dimensional structures or the microencapsulated ASC of the invention or ASC dissociated therefrom can be used for preventing, treating or ameliorating one or more symptoms associated with disease conditions. These include but are not limited to wound healing, tissue damage, allergic response, immune disease, autoimmune disease, immunologically mediated diseases, inflammatory disease, chronic inflammatory disease. Said use constitutes an additional aspect of the present invention.

Thus, in another aspect, the microencapsulated ASC of the invention or ASC dissociated therefrom are used as a medicament. In a particular embodiment, medicaments comprising of the microencapsulated ASC of the invention or ASC dissociated therefrom may be used for inducing transplantation tolerance, or for treating, and thereby alleviating, symptoms of autoimmune or inflammatory disorders, or immunologically mediated diseases including rejection of transplanted organs and tissues, in a subject suffering from any of said disorders or diseases. Thus, the microencapsulated ASC of the invention or ASC dissociated therefrom can be used to therapeutically or prophylactically treat and thereby alleviate symptoms of immune, autoimmune or inflammatory disorders in a subject suffering from any of said disorders or to alleviate symptoms of immunologically mediated diseases in a subject suffering from said diseases.

The microencapsulated ASC of the invention or ASC dissociated therefrom are of use in the treatment of autoimmune disease, inflammatory disorder or immunological mediated disease. Illustrative, non- limiting examples of said diseases and disorders which can be treated are those previously listed under heading "Definitions". In a particular embodiment, said inflammatory disease is a chronic inflammatory disease, such as, e.g., Celiac Disease, Multiple Sclerosis, Psoriasis, IBD or RA. In another aspect, the present invention relates to the use of the microencapsulated ASC of the invention or ASC dissociated therefrom for the preparation of a medicament for preventing, treating or ameliorating one or more symptoms associated with disorders in which modulation of a subject's immune system is beneficial, including, but not limited to, autoimmune diseases, inflammatory disorders, and immunologically mediated diseases including rejection of transplanted organs and tissues. Thus, the invention further refers to the use of the microencapsulated ASC of the invention or ASC dissociated therefrom for the preparation of a medicament for suppressing the immune response, or for inducing transplantation tolerance, or for treating autoimmune diseases, or for treating inflammatory disorders. Examples of said autoimmune diseases and inflammatory diseases have been previously mentioned. In a particular embodiment, disease is an inflammatory disease, such as a chronic inflammatory disease, e g , Celiac Disease, Multiple Sclerosis, Psoriasis, IBD or RA.

### Kits of the invention

Microencapsulated ASC as provided in the kits of the invention are contained within a sealed container in an environment comprising essentially of air (preferably of medicinal quality) or carbon dioxide.

The invention is further described in the following examples, to which the present invention is by no means limited.

### EXAMPLES

### EXAMPLE 1

### Preparation of ASC

Adipose tissue was obtained by liposuction. A cannula with a blunt end was introduced into the subcutaneous space through a small periumbilical incision (less than 0.5 cm in diameter). The suction was performed by moving the cannula along the adipose tissue compartment located under the abdominal wall, thus aiding the mechanical disruption of the adipose tissue. To minimize the loss of blood, a saline and epinephrine solution was injected as a vasoconstriction agent. 80-100 ml of raw lipoaspirate cells were obtained from each patient using this procedure.

The lipoaspirate was then washed with a phosphate and saline solution (PBS). The adipose tissue was then disrupted by digestion of the extracellular matrix with type II collagenase in saline solution (5 mg/ml) at 37 degrees for 30 minutes to release the cellular fraction. The collagenase was inactivated by adding an equivalent volume of DMEM medium with 10% fetal bovine serum. The cellular suspension was centrifuged at 250 g for 10 minutes to obtain a cell deposit. The cells were resuspended in DMEM medium with 10% fetal bovine serum. NH4CI was added at a final concentration of 0.16M and the cell; incubated for 10 minutes at room temperature to induce the lysis of erythrocytes present. The suspension was centrifuged at 250-400 g and resuspended in DMEM-10% FBS with 1% ampicillin-streptomycin. Finally, the cells were inoculated at a rate of 20-30,000 cells per cm² onto dishes.

The cells were kept in culture for 20-24 hours at 37° in an atmosphere with 5% CO2. After 24 hours of incubation, the dishes were washed with PBS to eliminate the cells that had not adhered and cellular remains.

### EXAMPLE 2

### Flow Focusing

Experiments were carried out to determine optimal pressure and flow-rate conditions for obtaining microparticles of uniform size of about 50-250 µm from 1% w/v alginate solutions. The experiments were carried out with Protanal LF 10/60 (FMC), Protanal LF 10/60S LS (FMC) and FLUKA alginate, all of them at 15 w/v. The viscosity and surface tension of the different solutions is shown in Table 1.

**Table 1: Physicochemical properties of the starting sodium alginate solutions**

| Sodium alginate | Concentration (% w/v) | Viscosity µ (cP) | Surface tension σ (mN/m) |
|---|---|---|---|
| Protanal LF 10/60 | 1 | 28,35 | 61,63 |
| Protanal LF 10/60 LS | 1 | 31,91 | 67,54 |
| FLUKA | 1 | 131,15 | 69,16 |

Flow focusing was carried out using an AVANT nebulizator having a body, a plate and a capilar. The body of the nebulizator comprises the chamber which is connected to a capillar through which the focused fluid enters the chamber and a tube of polymeric material through which the focusing fluid (pharmaceutical grade pressurized air) enters the chamber. The flow focusing is then carried out at different pressures of the focusing fluid and flow-rates of the focusing solution.

The size of the particles was determined by Image J and then compared to the predicted diameter. The results are shown in Table 2.

**Table 2: Particle size obtained using the different conditions of starting alginate, pressure of focusing fluid (ΔPt), flow rate of focusing solution (Qd), theoretical drop diameter (dg_th) and experimental drop diameter (dp)**

| Protanal LF 10/60 (1% w/v) | | | | Protanal LF 10/60 LS (1% w/v) | | | | FLUKA (1% w/v) | | | |
|---|---|---|---|---|---|---|---|---|---|---|---|
| ΔPt (mbar) | Qd | dg_th (µm) | dp (µm) | ΔPt (mbar) | Qd | dg_th (µm) | dp (µm) | ΔPt (mbar) | Qd | dg_th (µm) | dp (µm) |
| 40 | 60 | 203 | 87,5 | 40 | 60 | 206 | 85 | 50 | 35 | 182 | 60 |
| 20 | 65 | 273 | 121 | 20 | 65 | 266 | 122 | 50 | 60 | 244 | 81 |
| 20 | 100 | 316 | 163 | 20 | 100 | 322 | 149 | 30 | 90 | 315 | 203 |

### EXAMPLE 3

### Microencapsulation of ASC in alginate microparticles

ASC cells were released from the culture dishes, centrifuged and resuspended in DMEM-Hepes containing 5% human serum albumin (HSA) to a final concentration of 20% v/v. The cell suspension was then resuspended in 1,15% w/v polymeric alginate dispersion (PRONOVA UP LVG) and the mixture was added to a flow focusing device. The resulting microparticles were then stirred in a 3% w/v calcium chloride solution for 15 min. The concentration of cells in the sample was of 5.10⁶ cells/ml.

### EXAMPLE 4

### Microencapsulation of ASC in chitosan-coated microparticles

Cells prepared as in example 3 were then collected by centrifugation at 300 rpm and rinsed with PBS. The particles were incubated in a 0,5% w/v chitosan solution with shaking for 2 minutes.

### EXAMPLE 5

### Release of the ASC from the microparticles

Microparticles encapsulated with alginate in the presence of calcium were treated with a 2% w/v solution of citrate for a few seconds. This treatment proved effective in particles obtained using 0.1% w/v alginate (0,8% w/v final concentration) as well as in the particles obtained using 1,15% w/v (0,9% w/v final concentration).

Chitosan-coated microcapsules were treated with 2%, 3% and 5% w/v citrate solutions at different times (0', 2', 5', 10', 20' and 30'). ASC in chitosan-coated alginate particles could not be released in the presence of citrate even at the highest citrate concentrations and times of incubations. Cell viability could also not be assessed because Trypan Blue does not diffuse towards the interior of the particles.

### EXAMPLE 6

### Stability of the microencapsulated cells in chitosan-alginate or alginate

Cells microencapsulated in alginate obtained as in example 2 or cells encapsulated in alginate and coated with different concentrations of chitosan (0,1% w/v or 0,2% w/v) obtained as in example 3 were kept for up to 26 days at 22 °C at different concentrations of particles (30% v/v or 10% v/v) in an air atmosphere.

The microparticles were observed by optical microscopy in order to determine the shape and size of the particles, the efficency of cell encapsulation and the viability of microencapsulated cells. The determination of cell viability is carried out directly after the gelification step after collecting the particles by centrifugation and rinsing steps with PBS using Trypan Blue staining.

The percentages of viable cells at the different storage conditions are given in Tables 3 and 4.

**Table 3. Viability of encapsulated ASC (percentage viable cells) under different storage conditions. *Values not considered as they correspond to measurement errors.**

| | **CELL CONCENTRATION: 5x10⁶** | | | | | |
|---|---|---|---|---|---|---|
| Time (days) **v/v** | **CONDITIONS** | | | | | |
| | **AIR, 22°C** | | | | | |
| | **30% v/v (particle/medium)** | | | **10% v/v (particle/medium)** | | |
| | **0,1% w/v chitosan** | **0,2% w/v chitosan** | **No Chitosan** | **0,1% w/v chitosan** | **0,2% w/v chitosan** | **No Chitosan** |
| 1 | 91,56 | 92,32 | 95,47 | 79,54 | 93,26 | 94,13 |
| 2 | 86,97 | 86,48 | 88,49 | 77,59 | 89.12 | 92,48 |
| 3 | 77,94 | 88,71 | 82,56 | 81,15 | 88,63 | 86,75 |
| 5 | 74,74 | 82,13 | 73,18 | 75,94 | 84,31 | 87,13 |
| 7 | 64,3 | 66,67 | 63,15 | 59,25 | 57,87 | 74,86 |
| 10 | 62,04 | 82,15 | 34,87 | 11,21* | 7,14* | 64,02 |
| 15 | 22,15 | 53,27 | 41,92 | 56,24 | 29,30 | 43,12 |
| 19 | 39,4 | 29,34 | 25,86 | 57,14 | 41,34 | 39,36 |
| 26 | | | 18,97 | | | 22,15 |

**Table 4. Viability of encapsulated ASC (percentage viable cells) under different storage conditions.**

| | **CONDITIONS** | | | | | | | |
|---|---|---|---|---|---|---|---|---|
| | **Air** | | | | **CO₂** | | | |
| | **4°C** | | **22°C** | | **4°C** | | **22°C** | |
| Time (days) | **0.1% w/v Chitosan** | **No Chitosan** | **0.1% w/v Chitosan** | **No Chitosan** | **0.1% w/v Chitosan** | **No Chitosan** | **0.1% w/v Chitosan** | **No Chitosan** |
| 1 | 79,4 | 81,3 | 73,8 | 82,9 | 89 | 84,90 | 86,80 | *36,70* |
| 2 | 37,5 | 31,9 | 68,5 | 74,6 | 24,8 | 38,9 | 79,4 | 72,4 |
| 3 | 21,8 | 18,3 | 65,4 | 67,3 | 13,2 | 9,8 | 77,7 | 74,5 |
| 5 | 16,25 | 5,20 | 57 | 58,80 | 0 | 0 | 80,30 | 76,40 |
| 7 | 2 | 0 | 61,80 | 59,40 | 0 | 0 | 77,10 | 77,50 |
| 10 | 0 | | 46,70 | 43 | | | 69,2 | 63,7 |
| 14 | | | 43,10 | 38,70 | | | 59 | 46,70 |
| 19 | | | 47,10 | 43,70 | | | 61,70 | 42,30 |

The presence of chitosan results in an improvement on the cell viability when compared to cells which were not kept in chitosan.

The higher concentration of culture media (lower percentage of the particles/medium ratio) leads to a slight improvement in viability, in particular after 10 days of storage when the culture medium starts being limiting.

No substantial difference in viability was observed at the different cell concentrations tested. However, the presence of chitosan reduces the fragility of the microparticles associated to high cell concentrations. This effect is evident even at the lowest chitosan concentrations tested.

Comparing the different storage conditions, the best viability results are observed when the microcapsules are coated with 0.1% chitosan, contain high cell density and stored in a CO₂ atmosphere at a temperature of 22°C.

Temperature does not seem to affect the stability of the particle although it affects dramatically the cell viability. 100% of the cells encapsulated and stored at 4°C die at 7 days after the encapsulation. The cells stored at 22°C show a viability ratio of 60-75% in the same period and ca. 45% after 19 days.

The different types of atmosphere (air and CO₂) do not affect matrix stability although the cell survival in CO₂ is slightly higher, in particular during the first week when the medium is still not limiting.

The use of chitosan during the gelification step does not affect cell viability at short storage times. However, there exists a higher influence from 10 days onwards.

The use of a chitosan coat (0,1% w/v) seems to provide a slight advantage in comparison with the calcium ions coat with respect to the microparticle stability after long storage periods.

## Claims

1. A three-dimensional structure having a greatest dimension between 25 µm and 500 µm and comprising adipose derived stem cells and one or more adhesive agents, wherein the adhesive agent is a first cross-linked polymeric network, wherein the first cross-linked polymeric network is alginate, further comprising a second cross-linked polymeric network wherein said second cross-linked polymeric network encapsulates the first cross-linked network and wherein the second cross-linked polymeric network is chitosan.

2. A three-dimensional structure as defined in claim 1 wherein the adipose derived stem cells are embedded in said one or more adhesive agents.

3. A method for obtaining a 25-500 µm microparticle of adipose stem cells comprising the steps of
(i) combining a suspension of adipose stem cells and a solution of a cross-linkable polymer,
(ii) fragmenting the mixture obtained in step (i),
(iii) placing the fragments formed in step (ii) under conditions adequate for promoting the conversion of the cross-linkable polymer into a cross-linked polymeric network and
(iv) recovering the particles obtained in step (iii),
wherein the cross-linkable polymer is alginate, the method further comprising placing the microparticles obtained in step (iv) in the presence of a second cross-linkable polymer under conditions adequate for the cross-linking of said polymer, wherein the second cross-linkable polymer is chitosan.

4. A method as defined in claim 3 further comprising placing the microparticles under conditions adequate for removing the cross-links from said first polymeric network and, as the case may be, from said second polymeric network and for releasing the adipose stem cells.

5. A three-dimensional structure according to any of claims 1 or 2 further comprising a pharmaceutical carrier.

6. A three-dimensional structure according to any of claims 1 or 2 for use in medicine.

7. A three-dimensional structure according to any of claims 1 or 2 for use in the treatment of an inflammatory disease or an auto-immune disease.

8. A kit comprising a three-dimensional structure according to any of claims 1 or 2 in a CO₂ or air atmosphere.

## Patentansprüche

1. Dreidimensionale Struktur, die eine größte Dimension zwischen 25 µm und 500 µm aufweist und aus Körperfett abgeleitete Stammzellen und ein oder mehrere Haftmittel umfasst, wobei das Haftmittel ein erstes quervernetztes polymeres Netzwerk ist, wobei das erste quervernetzte polymere Netzwerk Alginat ist, weiter umfassend ein zweites quervernetztes polymeres Netzwerk, wobei besagtes zweites quervernetztes polymeres Netzwerk das erste quervernetzte Netzwerk einkapselt und wobei das zweite quervernetzte polymere Netzwerk Chitosan ist.

2. Dreidimensionale Struktur wie in Anspruch 1 definiert, wobei die aus Körperfett abgeleiteten Stammzellen in dem einen oder den mehreren Haftmitteln eingebettet sind.

3. Verfahren zur Bereitstellung eines 25-500 µm-Mikropartikels aus Fettstammzellen umfassend die Schritte
(i) Vereinigen einer Suspension aus Fettstammzellen und einer Lösung eines quervernetzbaren Polymers,
(ii) Fragmentierung der in Schritt (i) erhaltenen Mischung,
(iii) die in Schritt (ii) gebildeten Fragmente geeigneten Bedingungen aussetzen, um die Umwandlung des quervernetzbaren Polymers in ein quervernetztes polymeres Netzwerk zu befördern und
(iv) Rückgewinnung der in Schritt (iii) erhaltenen Partikel,
wobei das quervernetzbare Polymer Alginat ist und das Verfahren weiter umfasst, dass die in Schritt (iv) erhaltenen Mikropartikel in Gegenwart eines zweiten quervernetzbaren Polymers zur Quervernetzung des besagten Polymers geeigneten Bedingungen ausgesetzt werden, wobei das zweite quervernetzbare Polymer Chitosan ist.

4. Verfahren wie in Anspruch 3 definiert, das weiter umfasst, dass die Mikropartikel zur Entfernung der Quervernetzungen von besagtem ersten polymeren Netzwerk und, gegebenenfalls, von besagtem zweiten polymeren Netzwerk und zur Freisetzung der Fettstammzellen geeigneten Bedingungen ausgesetzt werden.

5. Dreidimensionale Struktur gemäß einem der Ansprüche 1 oder 2, weiter umfassend einen pharmazeutischen Trägerstoff.

6. Dreidimensionale Struktur gemäß einem der Ansprüche 1 oder 2 zur Verwendung in der Medizin.

7. Dreidimensionale Struktur gemäß einem der Ansprüche 1 oder 2 zur Verwendung bei der Behandlung inflammatorischer Erkrankungen oder einer Autoimmunerkrankung.

8. Kit umfassend eine dreidimensionale Struktur gemäß einem der Ansprüche 1 oder 2 in CO₂- oder Luftatmosphäre.

## Revendications

1. Une structure tridimensionnelle ayant une dimension maximale comprise entre 25 µm et 500 µm et comprenant des cellules souches dérivées adipeuses et un ou plusieurs agents adhésifs, l'agent adhésif étant un premier réseau polymère réticulé, le premier réseau polymère réticulé étant un alginate, comprenant en outre un deuxième réseau polymère réticulé, ledit deuxième réseau polymère réticulé encapsulant le premier réseau réticulé et le deuxième réseau polymère réticulé étant le chitosan.

2. La structure tridimensionnelle telle que définie dans la revendication 1, dans laquelle les cellules souches dérivées adipeuses sont noyées dans ledit un ou plusieurs agents adhésifs.

3. Un procédé d'obtention d'une microparticule de 25 à 500 µm de cellules souches adipeuses comprenant les étapes consistant à
(i) combiner une suspension de cellules souches adipeuses et une solution d'un polymère réticulable,
(ii) fragmenter le mélange obtenu à l'étape (i),
(iii) placer les fragments formés à l'étape (ii) dans des conditions adéquates pour favoriser la conversion du polymère réticulable en un réseau polymère réticulé et
(iv) récupérer les particules obtenues à l'étape (iii),
le polymère réticulable étant de l'alginate, le procédé comprenant en outre le fait de placer les microparticules obtenues à l'étape (iv) en présence d'un deuxième polymère réticulable dans des conditions adéquates pour la réticulation dudit polymère, le deuxième polymère réticulable étant le chitosan.

4. Un procédé tel que défini dans la revendication 3, comprenant en outre le fait de mettre en place des microparticules dans des conditions adéquates pour éliminer les réticulations dudit premier réseau polymère et, le cas échéant, dudit deuxième réseau polymère et pour libérer les cellules souches adipeuses.

5. Une structure tridimensionnelle selon l'une quelconque des revendications 1 ou 2 comprenant en outre un support pharmaceutique.

6. Une structure tridimensionnelle selon l'une quelconque des revendications 1 ou 2 pour une utilisation en médecine.

7. Une structure tridimensionnelle selon l'une quelconque des revendications 1 ou 2 pour une utilisation dans le traitement d'une maladie inflammatoire ou d'une maladie auto-immune.

8. Un kit comprenant une structure tridimensionnelle selon l'une quelconque des revendications 1 ou 2 dans une atmosphère de CO₂ ou d'air.
